(19) Europäisches Patentamt
European Patent Office
Office européen des brevets

(11) Publication number: **0 136 782 B1**

(12) **EUROPEAN PATENT SPECIFICATION**

(45) Date of publication of patent specification: **18.03.92** (51) Int. Cl.$^5$: **A61K 31/735**

(21) Application number: **84305221.8**

(22) Date of filing: **01.08.84**

(54) **Chondroitin sulfate/sodium hyaluronate compositions.**

(30) Priority: **09.08.83 US 521575**

(43) Date of publication of application:
**10.04.85 Bulletin 85/15**

(45) Publication of the grant of the patent:
**18.03.92 Bulletin 92/12**

(84) Designated Contracting States:
**CH DE FR GB IT LI SE**

(56) References cited:

**CHEMICAL ABSTRACTS, vol. 98, no. 19, 9th May 1983, page 44, no. 155193n, Columbus, Ohio, US; S.M. MAC RAE et al.: "The effects of sodium hyaluronate, chondroitin sulfate, and methylcellulose on the corneal endothelium and intraocular pressure", & AM. J. OPHTHALMOL. 1983, 95(3), 332-41**

(73) Proprietor: **NESTLE SA**
**Avenue Nestle 55**
**CH-1800 Vevey(CH)**

(72) Inventor: **Chang, Allison S.**
**7894 Fairview Road**
**Lesage West Virginia 25537(US)**
Inventor: **Boyd, James Edward**
**Route 1 P.O. Box 135**
**Barbourville West Virginia 25504(US)**
Inventor: **Koch, Harold Otto**
**1017 Big Bend Road**
**Barbourville West Virginia 25504(US)**
Inventor: **Johnson, Richard Michael**
**905 Woodbine Avenue**
**Rochester New York 14619(US)**

(74) Representative: **W.P. Thompson & Co.**
**Coopers Building, Church Street**
**Liverpool L1 3AB(GB)**

**EP 0 136 782 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to compositions obtained by mixing chondroitin sulfate or a sodium, potassium, magnesium or calcium salt thereof, and sodium, potassium, magnesium or calcium hyaluronate, together with an aqueous buffer, and the use thereof.

This invention relates to compositions for protecting both human and animal endothelial and epithelial cells which are subject to exposure to trauma. More particularly, this invention concerns compositions for protecting endothelial and epithelial cells in anticipation of surgical trauma using chondroitin sulfate/sodium hyaluronate compositions.

Since human corneal endothelial cells are not known to reproduce, it is of vital importance to protect endothelia to prevent cell damage prior to subjection to anticipated trauma, such as surgery. Recent advances in ophthalmic surgery have increased the need to protect corneal endothelial cells which may be subject to irreversible destruction during such surgery. Of particular significance is the need to protect corneal endothelial cells during intraocular lens (IOL) implantation, corneal transplantation and other intraocular surgical operations. Previous work in this field has been directed to protecting corneas with both non-biological and biological polymers.

Macromolecules heretofore employed in the protection of corneas include, separately, chondroitin sulfate and sodium hyaluronate. The use of a chondroitin sulfate solution for the protection of corneal surface tissue is described in a "CHONDRON®" product monogram, Kakan Pharmaceutical Company, Ltd., Tokyo, Japan, 1981. The use of sodium hyaluronate as an aid in ophthalmic surgery is described in a "HEALON®" product monogram, Pharmacia Laboratories, Piscataway, New Jersey, 1980. Solutions containing chondroitin sulfate or sodium hyaluronate alone have not met with complete satisfaction due to inadequate corneal dome maintenance which in turn provides spatial separation of corneal endothelium and surgical instruments, etc., or inadequate corneal endothelial cell protection, respectively.

In view of the above, it would be advantageous to prepare a viscous composition containing chondroitin sulfate and sodium hyaluronate, without the use of any other active material which could irritate or damage corneal endothelial cells.

Viscosity is normally a function of molecular weight at constant solute concentration. It has now been discovered that chondroitin sulfate and sodium hyaluronate may be mixed in aqueous buffer solution to produce a composition having surprisingly high viscosity and offering protection to corneal surface cells during intraocular lens implantation, corneal transplantation, and other intraocular surgical operations which is superior to that obtained with either of the individual active components.

According to the present invention there is provided an aqueous composition comprising an aqueous buffer to maintain a pH of 7 to 8, chondroitin sulfate or a sodium, potassium, magnesium or calcium salt thereof, and sodium, potassium, magnesium or calcium hyaluronate, the hyaluronate being used as a concentration of from 0.1 g up to 10 g in 100 mls. of water and the chondroitin sulfate or salt thereof being used as a concentration of from 0.1 g up to 10 g in 100 mls. of water.

A particularly useful composition is one wherein about 5.3 percent by weight of the total composition is chondroitin sulfate or salt thereof and about 4.2 percent by weight of the total solution is the hyaluronate.

Preferably the aqueous buffer includes monobasic sodium phosphate, dibasic sodium phosphate and sodium chloride mixed to maintain the pH of 7.0 to 8.0 and an osmolarity of 300 to 350 mOsmol/kg.

The compositions of the present invention may be used as active therapeutic materials. More particularly they may be used in protecting human or animal cell layers or tissue subject to exposure to trauma.

Additionally, the chondroitin sulfate/sodium hyaluronate compositions of the present invention can be administered after trauma as an aid in healing. Surprisingly, it has been found that the chondroitin sulfate/sodium hyaluronate compositions of the present invention exhibit enhanced solution stability and improved physical properties by comparison with the individual active components of the composition. These compositions are useful for topical applications as well as for irrigation during surgery.

Both chondroitin sulfate and sodium hyaluronate are glycosaminoglycans, commonly known as mucopolysaccharides. By mixing chondroitin sulfate and sodium hyaluronate in aqueous solution, it has been surprisingly found that the molecules appear to line up and attract each other by hydrogen bonding in the N-acetylamino group as shown below for a segment of molecular units. The hydrogen bonding interaction is only one of several possible interactions for chondroitin sulfate and sodium hyaluronate.

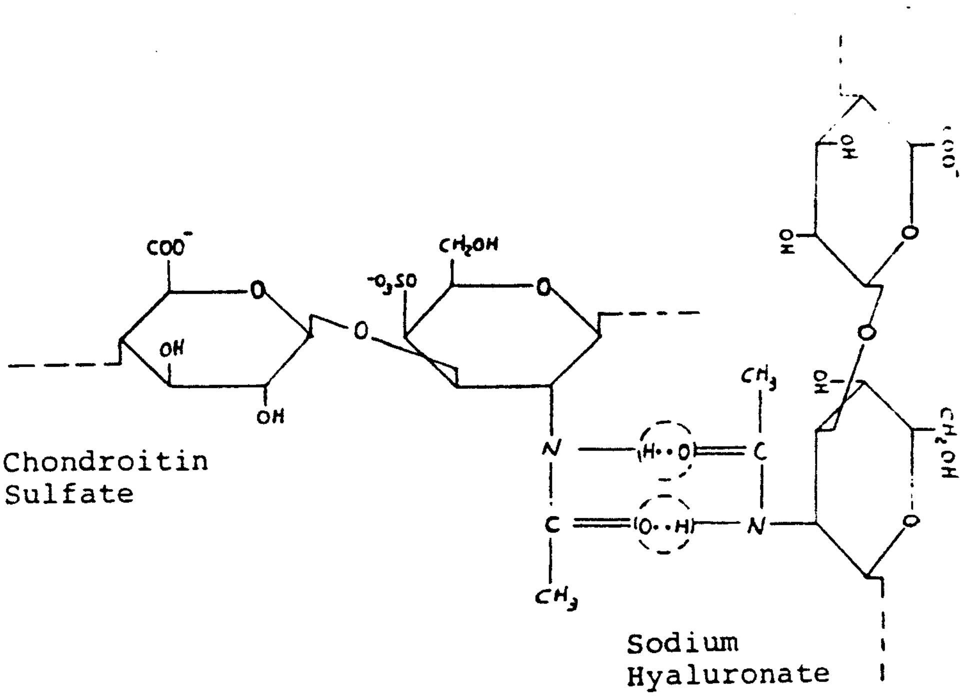


Turley and Roth, Nature, 283, pp. 268-271 (1980), have experimentally demonstrated that chondroitin sulfate-derivatized beads and hyaluronate-derivatized beads are capable of binding interaction with each other and have postulated that the interaction occurs between the carbohydrate chains of the polymers.

It has been surprisingly found that addition of chondroitin sulfate to sodium hyaluronate in aqueous solution or sodium hyaluronate to chondroitin sulfate in aqueous solution dramatically increases the viscosity of mixture. This increase in viscosity appears to be mainly due to increase in molecular weight rather than solute concentration increase. As those in the art are aware, viscosity of solute-solvent is a function of molecular weight and concentration of solute. The hydrogen bonding interaction postulated above between chondroitin sulfate and sodium hyaluronate would result in effectively enlarging the molecular size. Thus, viscosity of the mixture would be increased. The following examples demonstrate the nonlinear or synergistic change in physical properties by mixing of chondroitin sulfate and sodium hyaluronate in aqueous solution, which characterize the present invention. In the following examples, chondroitin sulfate is abbreviated CS, and sodium hyaluronate is abbreviated SH.

## Examples

A chondroitin sulfate/sodium hyaluronate solution was prepared by adding 5.3 grams of chondroitin sulfate to 4.2 grams of sodium hyaluronate in 100 ml of water containing 0.15 grams of monobasic sodium phosphate and 0.45 grams of dibasic sodium phosphate with a trace of sodium chloride. The viscosities of the mixture and individual solutions are shown below.

### Solution A

5.3 g CS/4.2 SH in 100 ml water with buffer.
Viscosity at 25 ° C = 71500 mPa s (71500 CPS) at shear rate less than 2/s.

### Solution B

4.2 g SH in 100 ml water with buffer.
Viscosity at 25 ° C = 58700 mPa s (58700 CPS) at shear rate less than 2/s.

### Solution C

5.3 g CS in 100 ml water with buffer.

Viscosity at 25°C = 10 mPa s (10 CPS)

A negative reaction (i.e., lack of synergistic change) of chondroitin sulfate with methyl cellulose (MC) is demonstrated by the following example. Two grams of chondroitin sulfate is added to 2 grams of methyl cellulose in 100 ml of water.

The viscosities of mixture and individual solutions are shown below.

Solution D

2 g MC/2 g CS*in 100 ml water.
Viscosity at 25°C = 5991 mPa s (5991 CPS) at shear rate less than 50 s.

Solution E

2 g MC in 100 ml water.
Viscosity at 25°C = 5857 mPa s (5857 CPS) at shear rate less than 50 s.

Solution F

2 g CS in 100 ml water.
Viscosity at 25°C = 3.0 mPa s (3.0 CPS).

Due to the molecular structure of methyl cellulose, the -N-C- groups in chondroitin sulfate could not possibly form hydrogen bonds with methyl cellulose. Methyl cellulose lacks -N-C- groups in the molecules. Hence, addition of chondroitin sulfate in methyl cellulose solution results in insignificant increase in viscosity. The observed small increase in viscosity is due to concentration effect only.

Interaction between chondroitin sulfate and sodium hyaluronate is believed to take place at any concentration. However, the synergistic viscosity effect increases with increasing concentration because of concentration effect and closeness of molecules for interaction. The solution of chondroitin sulfate/sodium hyaluronate mixture exhibits non-newtonian flow characteristics and has pseudo-plastic behaviour. The viscosity of pseudo-plastic substances decrease with increasing shear rates, i.e., relation of viscosity to shear rate is not linear. The following table shows the viscosity plotted against high shear rate at 25°C for solution "A".

| SHEAR RATES ($s^{-1}$) | | | | |
|---|---|---|---|---|
| | 250 | 500 | 2500 | 5000 |
| Absolute Viscosity, mPa•s (CPS) at 25°C | 1,774 | 1,075 | 307 | 181 |

Most recent data indicate that CS/SH compositions of the invention do not have a yield point.

In the practice of the invention sodium hyaluronate may be used at concentrations from 0.1 g up to 10 g in 100 ml water at temperatures from about 4°C to about 37°C. Chondroitin sulfate is also used at concentrations from 0.1 g up to 10 g in 100 ml water at temperatures from about 4°C to about 37°C. Within the ranges just described, any quantity of chondroitin sulfate can be added to form binding interaction with hyaluronate and produce physical and flow properties suitable for specific pharmaceutical and ophthalmic uses. Adding 12.6 g of chondroitin sulfate to 10 g sodium hyaluronate in water, the resulting solution has viscosity of over 1 million centipoises at 25°C (for low shear rate below 50 $s^{-1}$). A particularly useful composition is one wherein substantially 5.3 percent by weight of the total composition is chondroitin sulfate and substantially 4.2 percent by weight of the total composition is sodium hyaluronate.

The aqueous buffer solution used in the practice of the invention may be, for example, monobasic sodium phosphate, dibasic sodium phosphate, and sodium chloride mixed to form an aqueous buffer to maintain pH of 7 to 8.0 and osmoloarity of 300 to 350 mOsmol/kg. By raising the buffer concentration of monobasic sodium phosphate and dibasic sodium phosphate, the ionic strength of chondroitin sulfate/hyaluronate solution is increased. The kinetic rate constant of molecule interaction between chondroitin sulfate and hyaluronate is increased by raising ionic strength and temperature. This invention comprises concentrations of dibasic sodium phosphate and monobasic sodium phosphate from 0.1 g/100 ml to 5 g/100 ml and pH range of 7.0 to 8.0 at reaction temperatures between 4°C and 40°C. The following

*CS is insoluble in solution containing over 2 g MC.

example shows the effect of buffer on the viscosity or molecular weight of complex molecules for 5.3 g CS/4.2 g SH in 100 ml water:

Buffer 1: Dibasic sodium phosphate - 4.5 mg/ml
Sodium dihydrogen phosphate hydrate - 1.5 mg/ml
Viscosity of compositions of the present invention at 1 second and 25 °C is 68878 mPa s (68878 cps).

Buffer 2: Dibasic sodium phosphate - 7.5 mg/ml
Sodium dihydrogen phosphate hydrate - 1.0 mg/ml
Viscosity of compositions of the present invention at 1 second and 25 °C is 115011 mPa s (115011 cps).

The solution of chondroitin sulfate/hyaluronate mixture not only exhibits viscoelastic but also rheopectic behavior (i.e., viscosity increases with time at constant shear rate). At constant shear rate of 100 $s^{-1}$ for solution A, shear stress increases from 435 pascals to 452 pascals in 3 minutes. Both chondroitin sulfate and hyaluronate are helical straight chain molecules. In highly viscous environments the lower the temperature the less mobility both molecules have to align the N-acetylamine groups for mutual interaction. This interaction may take place at a very slow rate. However, the mutual interaction increases with raising of the temperature due to kinetic rate increase and better mobility of molecules to align the N-acetylamine groups for hydrogen bonding. When shear energy is added to the mixture, the kinetic energy of the molecules increases and the molecules are more easily aligned or oriented for bonding interaction. Thus, the viscosity is increased when shear energy is added to the mixture, and/or the mixture is subjected to increase in temperature, resulting in high molecular weight material.

Although the invention has been described in detail with reference to chondroitin sulfate and sodium hyaluronate it is also applicable to sodium, potassium, magnesium and calcium chondroitin sulfate and to potassium, magnesium and calcium hyaluronate.

**Claims**

**1.** Aqueous composition comprising an aqueous buffer to maintain a pH of 7 to 8, chondroitin sulfate or a sodium, potassium, magnesium or calcium salt thereof, and sodium, potassium, magnesium or calcium hyaluronate, the hyaluronate being used as a concentration of from 0.1 g up to 10 g in 100 ml of water and the chondroitin sulfate or salt thereof being used as a concentration of from 0.1 g up to 10 g in 100 ml of water.

**2.** Composition according to claim 1, wherein about 5.3 percent by weight of the total composition is chondroitin sulfate or salt thereof and about 4.2 percent by weight of the total solution is the hyaluronate.

**3.** Composition according to claim 1 or 2, wherein the aqueous buffer includes monobasic sodium phosphate, dibasic sodium phosphate and sodium chloride mixed to maintain the pH of 7.0 to 8.0 and an osmolarity of 300 to 350 mOsmol/kg.

**4.** Composition according to any of claims 1 to 3, for use as an active therapeutic material.

**5.** Composition according to any of claims 1 to 4, for use in protecting human or animal cell layers or tissues subject to exposure to trauma.

**Revendications**

**1.** Composition aqueuse comprenant un tampon aqueux pour maintenir un pH de 7 à 8, du sulfate de chondroïtine ou un sel de sodium, potassium, magnésium ou calcium de celui-ci, et un hyaluronate de sodium, potassium, magnésium ou calcium, le hyaluronate étant utilisé à une concentration de 0,1 g à 10 g dans 100 ml d'eau et le sulfate de chondroïtine ou son sel étant utilisé à une concentration de 0,1 g à 10 g dans 100 ml d'eau.

**2.** Composition selon la revendication 1, dans laquelle le sulfate de chondroïtine ou son sel constitue environ 5,3 pour cent en poids de la composition totale et le hyaluronate constitue environ 4,2 pour cent en poids de la composition totale.

**3.** Composition selon la revendication 1 ou 2, dans laquelle le tampon aqueux comprend du phosphate monosodique, du phosphate disodique et du chlorure de sodium mélangés pour maintenir le pH de 7,0 à 8,0 et une osmolarité de 300 à 350 mOsm/kg.

**4.** Composition selon l'une quelconque des revendications 1 à 3, pour son utilisation comme substance thérapeutique active.

**5.** Composition selon l'une quelconque des revendications 1 à 4, pour son utilisation dans la protection de couches cellulaires ou tissus humains ou animaux susceptibles d'être soumis à un traumatisme.

**Patentansprüche**

**1.** Wässrige Zusammensetzung, umfassend einen wässrigen Puffer zum Aufrechterhalten eines pH von 7 bis 8, Chondroitinsulfat oder ein Natrium-, Kalium-, Magnesium- oder Kalziumsalz davon und Natrium-, Kalium-, Magnesium- oder Kalziumhyaluronat, wobei das Hyaluronat bei einer Konzentration von 0,1 g bis zu 10 g in 100 ml Wasser verwendet wird und das Chondroitinsulfat oder das Salz davon in einer Konzentration von 0,1 bis zu 10 g in 100 ml Wasser verwendet wird.

**2.** Zusammensetzung nach Anspruch 1, dadurch **gekennzeichnet,** dass etwa 5,3 Gew.% der gesamten Zusammensetzung Chondroitinsulfat oder ein Salz davon und etwa 4,2 Gew.% der gesamten Lösung das Hyaluronat sind.

**3.** Zusammensetzung nach Anspruch 1 oder 2, dadurch **gekennzeichnet,** dass der wässrige Puffer monobasisches Natriumphosphat, dibasisches Natriumphosphat und Natriumchlorid umfasst, die vermischt sind, um den pH-Wert von 7,0 bis 8,0 und eine Osmolarität von 300 bis 350 mOsmol/kg aufrecht zu erhalten.

**4.** Zusammensetzung nach einem der Ansprüche 1 bis 3 zur Verwendung als ein aktives therapeutisches Material.

**5.** Zusammensetzung nach einem der Ansprüche 1 bis 4, zur Verwendung bei dem Schutz von menschlichen oder tierischen Zellschichten oder Geweben, die an einer Traumaexposition leiden.